# NEW EUROPEAN PATENT SPECIFICATION

(11) **EP 2 012 848 B2**
(45) Date of publication and mention of the opposition decision: **30.10.2024**
(45) Mention of the grant of the patent: 24.07.2019
(21) Application number: 07748068.9
(22) Date of filing: 26.04.2007
(51) Int. Cl.: A61M 1/14

(54) **REMOTE CONTROLLED MEDICAL APPARATUS**
FERNGESTEUERTE MEDIZINISCHE VORRICHTUNG
APPAREIL MEDICAL COMMANDE A DISTANCE

(30) Priority: 27.04.2006 SE 0600931; 30.05.2006 US 803447 P
(43) Date of publication of application: 14.01.2009
(73) Proprietor: Gambro Lundia AB, 220 10 Lund (SE)
(72) Inventor: GAGNER Johan, S-224 68 Lund (SE); MATTSSON Fredrik, S-168 50 Bromma (SE); HOBRO Sture, S-226-47 Lund (SE); NILSSON, Markus, s-260 34 Mörarp (SE); KLINTBERG Jonas, S-211 49 Malmö (SE)
(74) Representative: Ponzellini, Gian-Marco
(86) International application number: PCT/SE2007/000403
(87) International publication number: WO 2007/126360

(56) References cited:
- EP-A2- 1 574 178
- EP-A2- 1 574 178
- WO-A1-01/21065
- WO-A1-03/024322
- WO-A2-00/79466
- WO-A2-01/35577
- WO-A2-02/078783
- WO-A2-2005/044088
- DE-A1- 102004 011 264
- FR-A1- 2 806 561
- US-A- 5 151 082
- US-A- 5 401 238
- US-A- 5 544 649
- US-A- 6 039 251
- US-A1- 2001 027 384
- US-A1- 2002 082 728
- US-A1- 2002 103 453
- US-A1- 2002 103 453
- US-A1- 2004 088 027
- US-A1- 2004 088 027
- US-A1- 2005 055 244
- US-A1- 2005 055 244
- US-A1- 2005 055 244

## Description

### THE BACKGROUND OF THE INVENTION AND PRIOR ART

The present invention relates generally to a flexible and user-friendly blood cleaning treatment, which can be performed in non-hospital environments, such as in the patient's home. More particularly the invention relates to a dialysis unit according to the preamble of claim 1 and a medical system according to the preamble of claim 2.

The human body consists of approximately 60% water - a level which is important to maintain for survival. While it is unproble-matic to provide the body with new water, disposal of surplus water is a major problem in renal patients. One task of the normal kidney is to remove superfluous fluid from the blood, such as water, urea and other waste products. The resulting urine is transferred to the bladder and finally leaves the body during urination. The kidney's second task is to regulate for example the balance of electrolytes and acid and base. With malfunctioning kidneys, disorders may develop in most major body organs, a syndrome called uremia. If uremia remains untreated, it will lead to death. Uremia is treated by kidney transplantation or some form of blood cleaning, either extracorporeal (e.g. in the form of hemodialysis, hemofiltration or hemodiafiltration), or intracorporal (e.g. in the form of peritoneal dialysis).

Irrespective of which type of blood cleaning treatment that is used, the treatment normally requires a substantial amount of time; say three times per week in four hours per session. Thus, for a good patient comfort and quality of life, it is key that the treatments can be completed in a manner being as straightforward and flexible as possible. To this aim, various home dialysis solutions have been developed. Of course, a physician must supervise and analyze also these treatments. This can be accomplished by means of a smart card, which stores relevant therapy and treatment history data. The patient brings along his/her smart card when regularly visiting the clinic, so that the physician can study the treatment history data, and if necessary update the therapy prescription, which is also stored on the card. However, the smart card has a limited storage capacity, and therefore this solution requires that the patient visit the clinic rather frequently. An online connection between a central location (e.g. a hospital) and the home dialysis site may alleviate this problem.

US 2003/0001743 describes a personal and/or institutional health and wellness communications system, wherein a bi-directional communication is established over a network between a personal medical device and a central monitoring station. The medical device, which may be adapted to perform kidney dialysis, preferably communicates wirelessly with a local network node (e.g. according to the Bluetooth standard).

US 6,406,426 discloses a medical monitoring and alert system, which can be used with therapeutic devices, such as hemodialysis machines. Here, a connection is set up between a therapeutic device and a central monitoring system. This connection, which may include hardwired as well as wireless bi-directional links, enables patient information follow-ups, statistics, software updates and remote testing of the therapeutic device.

Naturally, the above-mentioned online connections between the remote host and the dialysis machine constitute improvements in relation to the smart card solution, for instance with regard to adjustments and testing of the dialysis machine as such. However still, a qualified caregiver must manually personalize the machine to meet the patient specific needs whenever the prescribed therapy is to be modified, and/or be adapted to the paward and flexible as possible. To this aim, various home dialysis solutions have been developed. Of course, a physician must supervise and analyze also these treatments. This can be accomplished by means of a smart card, which stores relevant therapy and treatment history data. The patient brings along his/her smart card when regularly visiting the clinic, so that the physician can study the treatment history data, and if necessary update the therapy prescription, which is also stored on the card. However, the smart card has a limited storage capacity, and therefore this solution requires that the patient visit the clinic rather frequently. An online connection between a central location (e.g. a hospital) and the home dialysis site may alleviate this problem.

US 2003/0001743 describes a personal and/or institutional health and wellness communications system, wherein a bi-directional communication is established over a network between a personal medical device and a central monitoring station. The medical device, which may be adapted to perform kidney dialysis, preferably communicates wirelessly with a local network node (e.g. according to the Bluetooth standard).

US 6,406,426 discloses a medical monitoring and alert system, which can be used with therapeutic devices, such as hemodialysis machines. Here, a connection is set up between a therapeutic device and a central monitoring system. This connection, which may include hardwired as well as wireless bi-directional links, enables patient information follow-ups, statistics, software updates and remote testing of the therapeutic device.

EP1 574 178 A2 discloses a medical treatment system with a data transmission connection for the transmission of audio and video between a treatment location and a doctor's location as well as exchange of treatment data between a computer of the treatment apparatus and of the doctor's location.

US 5,151,082 discloses an apparatus for kidney dialysis using in vivo separation of plasma from blood and extracorporeal separation of toxic metabolic waste from the separated plasma. The apparatus comprises a controller which accepts manual inputs and which may accept information from a remote dialysis center.

Naturally, the above-mentioned online connections between the remote host and the dialysis machine constitute improvements in relation to the smart card solution, for instance with regard to adjustments and testing of the dialysis machine as such. However still, a qualified caregiver must manually personalize the machine to meet the patient specific needs whenever the prescribed therapy is to be modified, and/or be adapted to the patient's current condition.

### SUMMARY OF THE INVENTION

The object of the present invention is therefore to solve the above problems and thus accomplish a solution by means of which a comparatively low degree of qualified caregiver intervention is required at the treatment site, and at the same time, the patient is relatively free to select the location at which the treatment is effected.

According to one aspect of the invention, the object is achieved by the initially described medical system, wherein the dialysis unit is adapted to influence the prescribed therapy in response to control data received from the remote host via the gateway unit during an ongoing treatment of the patient. Specifically, the dialysis unit is adapted to modify at least one parameter of the prescribed therapy in respect of the ongoing treatment before completing this treatment. Consequently, the dialysis process may be adapted in real time depending on how the patient responds to the treatment. An important advantage attained by this design is that any therapy updates and modifications can be effected smoothly and efficiently.

According to one preferred embodiment of this aspect of the invention, the control data defines at least one parameter of a prescribed therapy of a future treatment of the patient. Moreover, the dialysis unit is adapted to perform the future treatment in accordance with a prescribed therapy being adjusted with respect to the at least one parameter. Thus, based on the result of one treatment, a following therapy may be prescribed, either as a variation of the previous therapy, or as an entirely new therapy.

According to the invention, the dialysis unit is adapted to transmit at least one effect parameter to the remote host via the gateway unit. The effect parameter reflects a result of a treatment performed by the dialysis unit. Naturally, the effect parameter may provide information that aids the physician in his/her diagnosis work.

According to still another preferred embodiment of this aspect of the invention, the system includes a blood pressure monitor adapted to register at least one blood pressure related parameter in respect of the patient. The blood pressure monitor has a wireless interface towards the gateway unit, so that the monitor can transmit said at least one parameter to the gateway unit. The gateway unit, in turn, is adapted to transmit these records further to the remote host. Thereby, the remote host can gain valuable information about the patient's current condition, as well as how his/her health status develops during the treatment.

According to another preferred embodiment of this aspect of the invention, the dialysis unit is adapted to transmit at least one machine parameter to the remote host via the gateway unit. This at least parameter reflects a status, or a setting of, at least one characteristic of the dialysis unit. Thus, the remote host may log for example relevant pressures, fluid flows, temperatures, and settings of valves during the treatment. This aids the diagnosis, as well as facilitates the hardware maintenance and service.

According to yet another preferred embodiment of this aspect of the invention, the system includes a scale unit adapted to register a weight parameter in respect of the patient (typically the patient's body weight). Analogous to the above-mentioned blood pressure monitor, the scale unit has a wireless interface towards the gateway unit, and the scale unit is adapted to transmit the weight parameter to the gateway unit, which in turn, is adapted to transmit this data to the remote host. Consequently, the remote host can be informed about the patient's current weight, which is a vital factor for many diagnosis and therapy decisions. Moreover, if the scale unit is configured to register the weight parameter during the dialysis treatment, conclusions can be drawn regarding the water level balance based on how the weight fluctuates as the treatment progresses.

According to a further preferred embodiment of this aspect of the invention, the dialysis unit includes a memory module adapted to store data representing at least one executed treatment of the patient. The dialysis unit is also adapted to transmit at least a fraction of the stored data to the remote host via the gateway unit. Hence, the remote host can be informed of the outcome of previous therapies. Naturally, this is valuable information to the physician when prescribing new therapies.

According to yet another preferred embodiment of this aspect of the invention, the dialysis unit includes at least one software module adapted to control at least one function of the dialysis unit. Furthermore, the dialysis unit is adapted to receive software-updating data from the remote host via the gateway unit. In response to the software updating data, the dialysis unit is adapted to modify at least one of its software modules. Thus, the modus operandi of the dialysis unit can be modified from, or be determined by, the remote host.

According to another preferred embodiment of this aspect of the invention, the system includes a first data input unit (e.g. a personal digital assistant PDA), which is adapted to register manually entered information. The first data input unit has a wireless interface towards the gateway unit, and the data input unit is also adapted to transmit the manually entered information to the remote host via the gateway unit. Hence, the patient may enter subjective information, such as how he/she experienced the treatment and/or his/her current physical condition. Of course, this kind of information may be valuable for the physician when prescribing future therapies.

According to still another preferred embodiment of this aspect of the invention, the system includes a second data input unit (e.g. a bar code reader), which is adapted to automatically register machine-readable information. The second data input unit is also adapted to forward the machine-readable information to the remote host via the gateway unit. This transfer of data may be effected via the above-mentioned first data input unit, or directly to the gateway unit.

According to the invention the dialysis unit is adapted to influence the prescribed therapy in response to control data received from the remote host via the gateway unit during an ongoing treatment of the patient. Moreover, the dialysis unit is adapted to modify at least one parameter of the prescribed therapy in respect of the ongoing treatment before completing this treatment. Thus, any therapy updates and modifications of the treatment performed by the dialysis unit can be effected smoothly and efficiently.

The present disclosure concerns further the initially described method, wherein control data transmitted from the remote host are received in the gateway unit. The control data are then transmitted from the gateway unit to the dialysis unit over the wireless interface during an ongoing treatment of the patient. In response to the control data, at least one parameter of the prescribed therapy is modified in respect of the ongoing treatment before completing this treatment. The advantages of this method, as well as the preferred embodiments thereof, are apparent from the discussion hereinabove with reference to the proposed medical system.

Further, there is described a computer program directly loadable into the internal memory of a computer, comprising software for controlling the above proposed method when said program is run on a computer.

There is also described a computer readable medium, having a program recorded thereon, where the program is to make a computer control the above proposed method.

Thus, by means of the invention, a very high degree of treatment flexibility is attained. Namely, the patient can be treated in his/her home, or in any other suitable environment, and at the same time, the treatment can be monitored, and if necessary be adjusted from a remote location, such as a hospital.

Further advantages, advantageous features and applications of the present invention will be apparent from the following description and the dependent claims.

### BRIEF DESCRIPTION OF THE DRAWINGS

The present invention is now to be explained more closely by means of preferred embodiments, which are disclosed as examples, and with reference to the attached drawings.
- Figure 1: shows a block diagram over a medical system according to a one embodiment of the invention, and
- Figure 2: shows a flow diagram which illustrates the general method as further described.

### DESCRIPTION OF PREFERRED EMBODIMENTS OF THE INVENTION

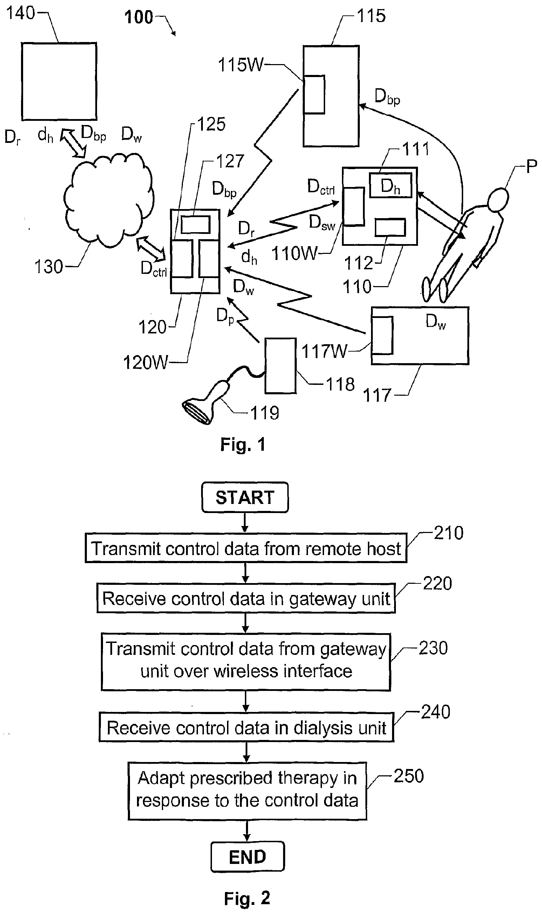

Figure 1 shows one embodiment of a proposed medical system 100 for cleaning the blood of a patient P under supervision of a central resource. The system 100 includes a dialysis unit 110, a gateway unit 120 and a remote host 140.

The dialysis unit 110 is adapted to treat the patient's P blood in accordance with a prescribed therapy by means of an extra- or an intracorporal treatment process. In any case, the dialysis unit 110 has a wireless interface 110W for bi-directional exchange of data, i.e. for receiving a first type of data D_{ctrl} and D_{sw} and for transmitting a second type of data Dᵣ and Dₕ. The wireless interface 110W is preferably adapted to the Bluetooth standard and/or Zigbee standard. Alternatively, the wireless interface 110W implements an infrared interface or it is based on another type of short-range wireless technology.

The gateway unit 120 has a matching wireless interface 120W, i.e. an interface that is adapted to communicate with the dialysis unit 110 over the interface 110W. Moreover, the gateway unit 120 has an interface 125, which is adapted to be connected to the remote host 140 via at least one interconnecting network 130. Depending on the interface format of a relevant access node to the network(s) 130, the interface 125 may be a wireless and/or a wire-bound interface. For example, if the access node is an Internet node, a PSTN node or a LAN node, the interface 125 is preferably wire-bound; whereas if the access node is a PLMN node or a WLAN node, the interface 125 is preferably wireless (PSTN = Public Switched Telephone Network; PLMN = Public Land Mobile Network (e.g. GSM, GPRS or 3G/UMTS); WLAN = Wireless Local-Area Network). Thus, by means of the interfaces 125 and 120W respectively, the gateway unit 120 is adapted to provide a bi-directional exchange of data between the remote host 140 and the dialysis unit 110. Specifically, the gateway unit 120 is adapted to receive therapy result Dᵣ; Dₕ and patient status data D_{bp} and D_{W} over the wireless interface 120W. The gateway unit 120 is also adapted to receive control data D_{ctrl} from the remote host 140 via the interface 125 and to forward the control data D_{ctrl} over the wireless interface 120W to the dialysis unit 110. Furthermore, the dialysis unit 110 is adapted to influence the prescribed therapy in response to the received control data D_{ctrl}.

The proposed gateway unit 120 is advantageous in that it standardizes the interface towards the dialysis unit, i.e. the gateway unit 120 renders the dialysis unit 110 independent from the format of any interconnecting networks 130. More importantly, however, the gateway unit 120 can ensure that the connection to the remote host 140 is protected against spoofing and eavesdropping, without requiring any security measures in the dialysis unit 110. Instead, the necessary encryption and authentication can be negotiated between the remote host 140 and the gateway unit 120. Of course, if the wireless interface 110W-120W is based on Bluetooth or Zigbee technology, the encryption and authentication available under these standards are preferably used to protect also the link between the gateway unit 120 and the dialysis unit 110.

According to one preferred embodiment of the invention, the dialysis unit 110 is adapted to receive the control data D_{ctrl} during an ongoing treatment of the patient P, and in response to the data D_{ctrl} modify at least one parameter of the prescribed therapy in respect of the ongoing treatment before completing this treatment.

The control data D_{ctrl} may also define at least one parameter of a prescribed therapy of a future treatment of the patient P (i.e. a treatment that has not yet been initiated). To this aim, the dialysis unit 110 includes a storage module (not shown), which is adapted to store the at least one parameter at least until said future treatment is to be completed. Of course, the dialysis unit 110 is then configured to perform the future treatment in accordance with a prescribed therapy that has been adjusted with respect to the at least one parameter. Dialysis liquid volumes, treatments times and UF (Ultra Filtration) values constitute examples of such parameters.

Depending on the type, number and extent of parameters included in the control data D_{ctrl}, this data may define up to an entire prescribed therapy of a future treatment of the patient P.

Since the wireless interface 110W is bi-directional, the dialysis unit 110 may also transmit uplink information to the gateway unit 120. For example, according to one preferred embodiment of the invention, the dialysis unit 110 is adapted to transmit at least one effect parameter Dᵣ to the gateway unit 120. The at least one effect parameter Dᵣ may include data representing a glucose concentration in the patient's P blood and/or a body water level.

The gateway unit 120, in turn, is adapted to forward the at least one effect parameter Dᵣ to the remote host 140. The effect parameters) Dᵣ reflect/s a result of a treatment performed by the dialysis unit 110. Hence, based on this/these parameter/s conclusions can be drawn at the remote host 140 whether or not the treatment was successful.

According to one preferred embodiment of the invention, the system 100 includes a blood pressure monitor 115, which is adapted to register at least one blood pressure related parameter in respect of the patient P (typically the diastolic pressure, the systolic pressure, the pulse and/or the medium arterial pressure, MAP). The monitor 115 has a wireless interface 115W towards the gateway unit 120, such that at least one of the at least one blood pressure related parameter D_{bp} can be transmitted to the remote host 140 via the gateway unit 120.

Preferably, the dialysis unit 110 is adapted to transmit at least one machine parameter Dₘ to the remote host 140 via the gateway unit 120. The at least one machine parameter Dₘ reflects a status for at least one characteristic of the dialysis unit 110, such as one or more pressure levels, various fluid flow rates, and/or liquid temperatures at different instances during the treatment. Alternatively, or as a complement thereto, the at least one machine parameter Dₘ may reflect a settings of various components, e.g. valves, in the dialysis unit 110. Thus, the remote host 140 may log the behavior of the dialysis unit 110. Consequently, the physician is aided in his/her diagnosis work. The hardware maintenance and service are also facilitated.

Preferably, the system 100 includes a scale unit 117, which is adapted to register a weight parameter D_{w} in respect of the patient P, such as the entire body weight. The scale unit 117 has a wireless interface 117W towards the gateway unit 120.

Thus, the scale unit 117 can transmit the weight parameter D_{w} to the remote host 140 via the gateway unit 120.

According to one preferred embodiment of the invention, the dialysis unit 110 may include, or be associated with, a memory module 111, which is adapted to store data Dₕ representing at least one executed treatment of the patient P (i.e. completed treatments as well as any treatments having been aborted before being completed). Furthermore, the dialysis unit 110 is adapted to transmit at least a fraction dₕ of the stored data Dₕ to the remote host 140 via the gateway unit 120. Hence, the remote host 140 can be informed of some or all characteristics of one or more earlier treatments.

According to one preferred embodiment of the invention, the system includes a first data input unit 118 (e.g. a PDA, a laptop or a smart phone), which is adapted to register manually entered information Dₚ. This information Dₚ relates to subjective data, such as how the patient experienced the treatment, or the patient's current physical condition. The first data input unit 118 has a wireless interface towards the gateway unit 120, and the unit 118 is adapted to transmit the manually entered information Dₚ to the remote host 140 via the gateway unit 120. Thereby, a physician at the remote host 140 can gain valuable information regarding the treatment, which may be helpful when prescribing future treatments. Additionally, the first data input unit 118 may be adapted to register event data, i.e. actions performed by the patient such as alarm acknowledgements or a premature ending of a treatment, and forward this data to the remote host 140 via the gateway unit 120.

It is also desirable if the system includes a second data input unit 119 (e.g. a bar code reader or a portable OCR scanner (OCR = Optical Character Recognition), which is adapted to automatically register machine readable information. Furthermore, the unit 119 is adapted to forward the machine-readable information to the remote host 140 via the gateway unit 120. This information transfer may either be effected via the first data input unit 118, as illustrated in Figure 1, or over the gateway unit 120 directly (for instance over the wireless interface 120W). By means of the second data input unit 119, the user may enter data pertaining to the dialysis fluid used, and thus provide the remote host with vital treatment information.

Preferably, the dialysis unit 110 includes, or is associated with, a computer readable medium 112, e.g. a memory module, which stores software for controlling the above-described functionality. The software, in turn, contains at least one software module that is adapted to control at least one function of the dialysis unit 110. Moreover, the dialysis unit 110 is adapted to receive software-updating data D_{sw} from the remote host 140 via the gateway unit 120. In response to the software updating data D_{sw}, the dialysis unit 110 is adapted to modify at least one of the at least one software modules. Thus, the modus operandi of the dialysis unit 110 can be altered/updated from the remote host 140.

In order to sum up, the general method will be described below with reference to the flow diagram in figure 2.

A first step 210 transmits control data D_{ctrl} from the remote host 140 to the gateway unit 120 (i.e. over the at least one interconnecting network 130). A step 220 then receives the control data in the gateway unit 120. Subsequently, a step 230 transmits the control data D_{ctrl} from the gateway unit 120 to the dialysis unit 110 over the wireless interface 120W-110W. Thereafter, a step 240 receives the control data D_{ctrl} in the dialysis unit 110. Finally, the prescribed therapy to be performed by the dialysis unit 110 is adapted in response to the received control data D_{ctrl}.

Preferably, the method also involves transmitting an acceptance message from the dialysis unit 110 over the wireless interface to the gateway unit (e.g. between steps 240 and 250). The acceptance message acknowledges reception of the control data D_{ctrl} in the dialysis unit 110. It is further preferable if the gateway unit 120 is adapted to retransmit the control data D_{ctrl} to the dialysis unit 110 until such an acceptance message has been received. Moreover, upon receipt of the acceptance message, the dialysis unit 110 is preferably adapted to forward the acceptance message (or any equivalent message) to the remote host 140. Thereby, the remote host 140 can be informed of the fact that the prescribed therapy and/or the modus operandi the dialysis unit 110 will be updated as desired.

All of the process steps, as well as any sub-sequence of steps, described with reference to the figure 2 above may be controlled by means of a programmed computer apparatus. The program may be in the form of source code; object code, a code intermediate source and object code such as in partially compiled form, or in any other form suitable for use in the implementation of the process according to the invention. The carrier may be any entity or device capable of carrying the program. For example, the carrier may comprise a storage medium, such as a Flash memory, a ROM (Read Only Memory), for example a CD (Compact Disc) or a semiconductor ROM, an EPROM (Erasable Programmable Read-Only Memory), an EEPROM (Electrically Erasable Programmable Read-Only Memory), or a magnetic recording medium, for example a floppy disc or hard disc. Further, the carrier may be a transmissible carrier such as an electrical or optical signal which may be conveyed via electrical or optical cable or by radio or by other means. When the program is embodied in a signal which may be conveyed directly by a cable or other device or means, the carrier may be constituted by such cable or device or means. Alternatively, the carrier may be an integrated circuit in which the program is embedded, the integrated circuit being adapted for performing, or for use in the performance of, the relevant processes.

The term "comprises/comprising" when used in this specification is taken to specify the presence of stated features, integers, steps or components. However, the term does not preclude the presence or addition of one or more additional features, integers, steps or components or groups thereof.

The reference to any prior art in this specification is not, and should not be taken as, an acknowledgement or any suggestion that the referenced prior art forms part of the common general knowledge in Australia.

The invention is not restricted to the described embodiments in the figures, but may be varied freely within the scope of the claims.

## Claims

1. A dialysis unit (110) adapted to treat the patient's (P) blood in accordance with a prescribed therapy, the dialysis unit (110) having a first wireless interface (110W) for bi-directional exchange of data (D_{ctrl}, D_{sw}, Dᵣ, Dₕ), and the dialysis unit (110) being adapted to communicate with a remote host (140) via a gateway unit (120) having a second wireless interface (120W) matched to the first wireless interface (110W), gateway unit (120) being further connected to the remote host (140) via at least one interconnecting network (130), the dialysis unit (110) being adapted to transmit at least one effect parameter (Dᵣ), the at least one effect parameter (Dᵣ) reflecting a result of a treatment performed by the dialysis unit (110) to the remote host via the gateway unit (120) during the treatment, **characterized in that** the dialysis unit (110) is further adapted to:
influence the prescribed therapy in response to control data (D_{ctrl}) received from the remote host (140) via the gateway unit (120), the control data (D_{ctrl}) being received during an ongoing treatment of the patient (P), and
modify at least one parameter of the prescribed therapy in respect of the ongoing treatment before completing this treatment.

2. A medical system for cleaning the blood of a patient (P), the system (100) comprising:
a dialysis unit (110) according to claim 1, and
the gateway unit (120) adapted to communicate with the dialysis unit (110) over the first and second wireless interfaces (110W, 120W), the gateway unit (120) having an interface (125) adapted to be connected to the remote host (140) via the at least one interconnecting network (130), and the gateway unit (120) being adapted to provide a bi-directional exchange of data between the remote host (140) and the dialysis unit (110), **characterized in that** the dialysis unit (110) is adapted to:
transmit the at least one effect parameter (Dᵣ) to the remote host via the gateway unit (120) during the treatment; and
influence the prescribed therapy in response to control data (D_{ctrl}) received from the remote host (140) via the gateway unit (120), the control data (D_{ctrl}) being received during an ongoing treatment of the patient (P), and
modify at least one parameter of the prescribed therapy in respect of the ongoing treatment before completing this treatment.

3. The system according to claim 2, wherein the control data (D_{ctrl}) defines at least one parameter of a prescribed therapy of a future treatment of the patient (P), and the dialysis unit (110) is adapted to perform the future treatment in accordance with a prescribed therapy being adjusted with respect to the at least one parameter.

4. The system according to claim 3, wherein the control data (D_{ctrl}) defines an entire prescribed therapy of a future treatment of the patient (P).

5. The system according to any one of claim 2 to claim 4, wherein the system (100) comprises a blood pressure monitor (115) adapted to register at least one blood pressure related parameter in respect of the patient (P), the blood pressure monitor (115) having a wireless interface (115W) towards the gateway unit (120), and the blood pressure monitor (115) being adapted to transmit at least one of the at least one blood pressure related parameter (D_{bp}) to the remote host (140) via the gateway unit (120).

6. The system according to any one of claim 2 to claim 5, wherein the dialysis unit (110) is adapted to transmit at least one machine parameter (Dₘ) to the remote host (140) via the gateway unit (120), the at least one machine parameter (Dₘ) reflecting a status or a setting of at least one characteristic of the dialysis unit (110).

7. The system according to any one of claim 2 to claim 6, wherein the system (100) comprises a scale unit (117) adapted to register a weight parameter (D_{w}) in respect of the patient (P), the scale unit (117) having a wireless interface (117W) towards the gateway unit (120), and the scale unit (117) being adapted to transmit the weight parameter (D_{w}) to the remote host (140) via the gateway unit (120).

8. The system according to any one of claim 2 to claim 7, wherein the dialysis unit (110) comprises a memory module (111) adapted to store data (Dₕ) representing at least one executed treatment of the patient (P), and the dialysis unit (110) is adapted to transmit at least a fraction (dₕ) of the stored data (Dₕ) to the remote host (140) via the gateway unit (120).

9. The system according to any one of claim 2 to claim 8, wherein the dialysis unit (110) comprises at least one software module adapted to control at least one function of the dialysis unit (110), and the dialysis unit (110) is adapted to:
receive software updating data (D_{sw}) from the remote host (140) via the gateway unit (120), and
modify at least one of the at least one software module in response to the software updating data (D_{sw}).

10. The system according to any one of claim 2 to claim 9, comprising a first data input unit (118) adapted to register manually entered information (Dₚ), the first data input unit (118) having a wireless interface towards the gateway unit (120), and the data input unit (118) being adapted to transmit the manually entered information (Dₚ) to the remote host (140) via the gateway unit (120).

11. The system according to any one of claim 2 to claim 10, comprising a second data input unit (119) adapted to:
automatically register machine readable information, and
forward the machine readable information to the remote host (140) via the gateway unit (120).

## Patentansprüche

1. Dialyseeinheit (110), die eingerichtet ist, um das Blut eines Patienten (P) gemäß einer verordneten Therapie zu behandeln, wobei die Dialyseeinheit (110) eine erste Drahtlosschnittstelle (110W) zum bidirektionalen Austausch von Daten (D_{ctrl}, D_{sw}, Dᵣ, Dₕ) aufweist, und die Dialyseeinheit (110) eingerichtet ist, um mit einem entfernt befindlichen Host (140) über die Gateway-Einheit (120) zu kommunizieren, die eine zweite Drahtlosschnittstelle (120W) aufweist, die auf die erste Drahtlosschnittstelle (110W) abgestimmt ist, wobei die Gateway-Einheit (120) ferner über mindestens ein Verbindungsnetzwerk (130) mit dem entfernt befindlichen Host (140) verbunden ist, wobei die Dialyseeinheit (110) eingerichtet ist, um mindestens einen Effektparameter (Dᵣ) zu übertragen, wobei der mindestens eine Effektparameter (Dᵣ) ein Ergebnis einer Behandlung, die durch die Dialyseeinheit (110) durchgeführt wird, während der Behandlung über die Gateway-Einheit (120) an den entfernt befindlichen Host reflektiert, **dadurch gekennzeichnet, dass** die Dialyseeinheit (110) ferner eingerichtet ist zum:
Beeinflussen der verordneten Therapie in Reaktion auf Steuerdaten (D_{ctrl}), die über die Gateway-Einheit (120) von dem entfernt befindlichen Host (140) empfangen werden, wobei die Steuerdaten (D_{ctrl}) während einer laufenden Behandlung des Patienten (P) empfangen werden, und
Modifizieren mindestens eines Parameters der verordneten Therapie in Bezug auf die laufende Behandlung vor dem Abschluss dieser Behandlung.

2. Medizinisches System zum Reinigen des Blutes eines Patienten (P), das System (100) umfassend:
eine Dialyseeinheit (110) nach Anspruch 1, und
die Gateway-Einheit (120), die eingerichtet ist, um mit der Dialyseeinheit (110) über die ersten und zweiten Drahtlosschnittstellen (110W, 120W) zu kommunizieren, wobei die Gateway-Einheit (120) eine Schnittstelle (125) aufweist, die eingerichtet ist, um über das mindestens ein Verbindungsnetzwerk (130) mit dem entfernt befindlichen Host (140) verbunden zu werden, und wobei die Gateway-Einheit (120) eingerichtet ist, um einen bidirektionalen Austausch von Daten zwischen dem entfernt befindlichen Host (140) und der Dialyseeinheit (110) bereitzustellen, **dadurch gekennzeichnet, dass** die Dialyseeinheit (110) eingerichtet ist zum:
Übertragen des mindestens einen Effektparameters (Dᵣ) an den entfernt befindlichen Host über die Gateway-Einheit (120) während der Behandlung; und
Beeinflussen der verordneten Therapie in Reaktion auf Steuerdaten (D_{ctrl}), die von dem entfernt befindlichen Host (140) über die Gateway-Einheit (120) empfangen werden, wobei die Steuerdaten (D_{ctrl}) während einer laufenden Behandlung des Patienten (P) empfangen werden, und
Modifizieren mindestens eines Parameters der verordneten Therapie in Bezug auf die laufende Behandlung vor dem Abschluss dieser Behandlung.

3. System nach Anspruch 2, wobei die Steuerdaten (D_{ctrl}) mindestens einen Parameter einer verordneten Therapie einer zukünftigen Behandlung des Patienten (P) definieren, und die Dialyseeinheit (110) eingerichtet ist, um die zukünftige Behandlung gemäß einer verordneten Therapie durchzuführen, die in Bezug auf den mindestens einen Parameter angepasst worden ist.

4. System nach Anspruch 3, wobei die Steuerdaten (D_{ctrl}) eine gesamte verordnete Therapie einer zukünftigen Behandlung des Patienten (P) definieren.

5. System nach einem der Ansprüche 2 bis 4, wobei das System (100) einen Blutdruckmonitor (115) umfasst, der eingerichtet ist, um mindestens einen blutdruckbezogenen Parameter in Bezug auf den Patienten (P) zu registrieren, wobei der Blutdruckmonitor (115) eine Drahtlosschnittstelle (115W) zu der Gateway-Einheit (120) aufweist, und der Blutdruckmonitor (115) eingerichtet ist, um mindestens einen von dem mindestens einen blutdruckbezogenen Parameter (D_{bp}) über die Gateway-Einheit (120) an den entfernt befindlichen Host (140) zu übertragen.

6. System nach einem von Anspruch 2 bis Anspruch 5, wobei die Dialyseeinheit (110) eingerichtet ist, um über die Gateway-Einheit (120) mindestens einen Maschinenparameter (Dₘ) an den entfernt befindlichen Host (140) zu übertragen, wobei der mindestens eine Maschinenparameter (Dₘ) einen Status oder eine Einstellung von mindestens einem Charakteristikum der Dialyseeinheit (110) reflektiert.

7. System nach einem von Anspruch 2 bis Anspruch 6, wobei das System (100) eine Waageneinheit (117) umfasst, die eingerichtet ist, um einen Gewichtsparameter (D_{w}) in Bezug auf den Patienten (P) zu registrieren, wobei die Waageneinheit (117) eine Drahtlosschnittstelle (117W) zu der Gateway-Einheit (120) aufweist, und wobei die Waageneinheit (117) eingerichtet ist, um den Gewichtsparameter (D_{w}) über die Gateway-Einheit (120) an den entfernt befindlichen Host (140) zu übertragen.

8. System nach einem von Anspruch 2 bis Anspruch 7, wobei die Dialyseeinheit (110) ein Speichermodul (111) umfasst, das eingerichtet ist, um Daten (Dₕ) zu speichern, die mindestens eine ausgeführte Behandlung des Patienten (P) repräsentieren, und wobei die Dialyseeinheit (110) eingerichtet ist, um mindestens eine Fraktion (dₕ) der gespeicherten Daten (Dₕ) über die Gateway-Einheit (120) an den entfernt befindlichen Host (140) zu übertragen.

9. System nach einem von Anspruch 2 bis Anspruch 8, wobei die Dialyseeinheit (110) mindestens ein Softwaremodul umfasst, das eingerichtet ist, um mindestens eine Funktion der Dialyseeinheit (110) zu steuern, und wobei die Dialyseeinheit (110) eingerichtet ist zum:
Empfangen von Software-Aktualisierungsdaten (D_{sw}) von dem entfernt befindlichen Host (140) über die Gateway-Einheit (120), und
Modifizieren von mindestens einem des mindestens einen Softwaremoduls in Reaktion auf die Software-Aktualisierungsdaten (D_{sw}) .

10. System nach einem von Anspruch 2 bis Anspruch 9, umfassend eine erste Dateneingabeeinheit (118), die eingerichtet ist, um manuell eingegebene Informationen (Dₚ) zu registrieren, wobei die erste Dateneingabeeinheit (118) eine Drahtlosschnittstelle zu der Gateway-Einheit (120) aufweist, und wobei die Dateneingabeeinheit (118) eingerichtet ist, um die manuell eingegebenen Informationen (Dₚ) über die Gateway-Einheit (120) an den entfernt befindlichen Host (140) zu übertragen.

11. System nach einem von Anspruch 2 bis Anspruch 10, umfassend eine zweite Dateneingabeeinheit (119), die eingerichtet ist zum:
automatischen Registrieren von maschinenlesbaren Informationen, und
Weiterleiten der maschinenlesbaren Informationen an den entfernt befindlichen Host (140) über die Gateway-Einheit (120).

## Revendications

1. Unité de dialyse (110) adaptée pour traiter le sang d'un patient (P) selon une thérapie prescrite, l'unité de dialyse (110) ayant une première interface sans fil (110W) pour un échange bidirectionnel de données (D_{ctrl}, D_{sw}, Dᵣ, Dₕ), et l'unité de dialyse (110) étant adaptée pour communiquer avec un hôte distant (140) par le biais d'une unité passerelle (120) ayant une deuxième interface sans fil (120W) couplée à la première interface sans fil (110W), l'unité passerelle (120) étant également connectée à l'hôte distant (140) par le biais d'au moins un réseau d'interconnexion (130), l'unité de dialyse (110) étant adaptée pour transmettre au moins un paramètre d'effet (Dᵣ), l'au moins un paramètre d'effet (Dᵣ) reflétant un résultat d'un traitement effectué par l'unité de dialyse (110), à l'hôte distant par le biais de l'unité passerelle (120) pendant le traitement, **caractérisée en ce que** l'unité de dialyse (110) est également adaptée pour :
influencer la thérapie prescrite en réponse à des données de contrôle (D_{ctrl}) reçues depuis l'hôte distant (140) par le biais de l'unité passerelle (120), les données de contrôle (D_{ctrl}) étant reçues pendant un traitement en cours du patient (P), et
modifier au moins un paramètre de la thérapie prescrite concernant le traitement en cours avant la fin de ce traitement.

2. Système médical destiné à nettoyer le sang d'un patient (P), le système (100) comprenant :
une unité de dialyse (110) selon la revendication 1, et
l'unité passerelle (120) adaptée pour communiquer avec l'unité de dialyse (110) sur les première et deuxième interfaces sans fil (110W, 120W), l'unité passerelle (120) ayant une interface (125) adaptée pour être connectée à l'hôte distant (140) par le biais de l'au moins un réseau d'interconnexion (130), et l'unité passerelle (120) étant adaptée pour assurer un échange bidirectionnel de données entre l'hôte distant (140) et l'unité de dialyse (110), **caractérisé en ce que** l'unité de dialyse (110) est adaptée pour :
transmettre l'au moins un paramètre d'effet (Dᵣ) à l'hôte distant par le biais de l'unité passerelle (120) pendant le traitement ; et
influencer la thérapie prescrite en réponse à des données de contrôle (D_{ctrl}) reçues depuis l'hôte distant (140) par le biais de l'unité passerelle (120), les données de contrôle (D_{ctrl}) étant reçues pendant un traitement en cours du patient (P), et
modifier au moins un paramètre de la thérapie prescrite concernant le traitement en cours avant la fin de ce traitement.

3. Système selon la revendication 2, dans lequel les données de contrôle (D_{ctrl}) définissent au moins un paramètre d'une thérapie prescrite d'un futur traitement du patient (P), et l'unité de dialyse (110) est adaptée pour effectuer le futur traitement conformément à une thérapie prescrite qui est ajustée par rapport à l'au moins un paramètre.

4. Système selon la revendication 3, dans lequel les données de contrôle (D_{ctrl}) définissent une thérapie prescrite complète d'un futur traitement du patient (P) .

5. Système selon l'une quelconque des revendications 2 à 4, dans lequel le système (100) comprend un moniteur de pression artérielle (115) adapté pour enregistrer au moins un paramètre associé à la pression artérielle concernant le patient (P), le moniteur de pression artérielle (115) ayant une interface sans fil (115W) vers l'unité passerelle (120), et le moniteur de pression artérielle (115) étant adapté pour transmettre au moins un de l'au moins un paramètre associé à la pression artérielle (D_{bp}) à l'hôte distant (140) par le biais de l'unité passerelle (120).

6. Système selon l'une quelconque des revendications 2 à 5, dans lequel l'unité de dialyse (110) est adaptée pour transmettre au moins un paramètre machine (Dₘ) à l'hôte distant (140) par le biais de l'unité passerelle (120), l'au moins un paramètre machine (Dₘ) reflétant un état ou un réglage d'au moins une caractéristique de l'unité de dialyse (110).

7. Système selon l'une quelconque des revendications 2 à 6, dans lequel le système (100) comprend une unité de pesée (117) adaptée pour enregistrer un paramètre pondéral (D_{w}) concernant le patient (P), l'unité de pesée (117) ayant une interface sans fil (117W) vers l'unité passerelle (120), et l'unité de pesée (117) étant adaptée pour transmettre le paramètre pondéral (D_{w}) à l'hôte distant (140) par le biais de l'unité passerelle (120).

8. Système selon l'une quelconque des revendications 2 à 7, dans lequel l'unité de dialyse (110) comprend un module de mémoire (111) adapté pour stocker des données (Dₕ) représentant au moins un traitement exécuté du patient (P), et l'unité de dialyse (110) est adaptée pour transmettre au moins une fraction (dₕ) des données stockées (Dₕ) à l'hôte distant (140) par le biais de l'unité passerelle (120).

9. Système selon l'une quelconque des revendications 2 à 8, dans lequel l'unité de dialyse (110) comprend au moins un module logiciel adapté pour contrôler au moins une fonction de l'unité de dialyse (110), et l'unité de dialyse (110) est adaptée pour :
recevoir des données d'actualisation de logiciel (D_{sw}) depuis l'hôte distant (140) par le biais de l'unité passerelle (120), et
modifier au moins un de l'au moins un module logiciel en réponse aux données d'actualisation de logiciel (D_{sw}) .

10. Système selon l'une quelconque des revendications 2 à 9, comprenant une première unité de saisie de données (118) adaptée pour enregistrer des informations saisies manuellement (Dₚ), la première unité de saisie de données (118) ayant une interface sans fil vers l'unité passerelle (120), et l'unité de saisie de données (118) étant adaptée pour transmettre les informations saisies manuellement (Dₚ) à l'hôte distant (140) par le biais de l'unité passerelle (120).

11. Système selon l'une quelconque des revendications 2 à 10, comprenant une deuxième unité de saisie de données (119) adaptée pour :
enregistrer automatiquement des informations lisibles par machine, et
transférer les informations lisibles par machine à l'hôte distant (140) par le biais de l'unité passerelle (120) .
